(19) Europäisches Patentamt
European Patent Office
Office européen des brevets

(11) EP 2 087 881 A1

(12) # DEMANDE DE BREVET EUROPEEN

(43) Date de publication:
**12.08.2009 Bulletin 2009/33**

(21) Numéro de dépôt: **09151295.4**

(22) Date de dépôt: **26.01.2009**

(51) Int Cl.:
***A61K 8/72*** *(2006.01)* ***A61Q 1/00*** *(2006.01)*
***C08J 3/09*** *(2006.01)* ***C08L 33/00*** *(2006.01)*

(84) Etats contractants désignés:
**AT BE BG CH CY CZ DE DK EE ES FI FR GB GR HR HU IE IS IT LI LT LU LV MC MK MT NL NO PL PT RO SE SI SK TR**
Etats d'extension désignés:
**AL BA RS**

(30) Priorité: **01.02.2008 FR 0850636**

(71) Demandeur: **L'Oréal**
**75008 Paris (FR)**

(72) Inventeur: **Farcet, Céline**
**75012, PARIS (FR)**

(74) Mandataire: **Dodin, Catherine**
**L'Oréal**
**River Plaza - D.I.P.I.**
**25-29 Quai Aulagnier**
**92665 Asnières-sur-Seine Cedex (FR)**

(54) **Dispersion de particules de polymère, composition la comprenant et procédé de traitement cosmétique**

(57) La présente demande concerne de nouvelles dispersions de particules de polymère stabilisé en surface, dans un milieu non aqueux, dans lesquelles le polymère comprend 5 à 50% en poids de monomères éthyléniques comprenant une partie PEG (polyéthylèneglycol).

L'invention a également pour objet une composition cosmétique ou pharmaceutique comprenant, dans un milieu cosmétiquement ou pharmaceutiquement acceptable, ladite dispersion. Cette composition peut en particulier être un rouge à lèvres ou une composition capillaire.

L'invention concerne encore un procédé de traitement cosmétique des matières kératiniques, employant ladite composition.

EP 2 087 881 A1

## Description

La présente invention a trait à une nouvelle dispersion de particules de polymères bien particuliers, dispersées dans un milieu non aqueux, ainsi qu'aux compositions notamment cosmétiques ou pharmaceutiques comprenant ladite dispersion.

Il est connu d'utiliser en cosmétique des dispersions de particules de polymère, généralement de taille nanométrique, dans des milieux organiques, et notamment, par EP749747, des dispersions non aqueuses de particules de poly(méth)acrylate de méthyle, dans une huile de paraffine non volatile ou dans l'isododécane, par exemple. Toutefois, ces dispersions ne permettent pas d'obtenir des propriétés cosmétiques optimales, notamment en terme de glissant, de confort, de tenue et de toucher, en particulier de douceur.

La demanderesse a découvert de manière surprenante de nouvelles dispersions de particules de polymères, stabilisées par des stabilisants, dans des milieux non aqueux, qui permettent d'apporter les propriétés cosmétiques recherchées (toucher, douceur, glissant).

L'invention a donc pour objet une dispersion de particules d'au moins un polymère éthylénique stabilisé en surface par un agent stabilisant, dans un milieu non aqueux comprenant au moins un composé non aqueux, liquide à 25°C, ayant un paramètre de solubilité global selon l'espace de solubilité de HANSEN inférieur ou égal à 20 $(MPa)^{1/2}$, ou un mélange de tels composés; **caractérisée en ce que** ledit polymère éthylénique comprend de 5% à 50% (exclu) en poids de monomère de formule (I) telle que définie ci-après, seul ou en mélange, par rapport au poids total de monomères.
L'invention a également pour objet une composition cosmétique ou pharmaceutique comprenant, dans un milieu cosmétiquement ou pharmaceutiquement acceptable, au moins une dispersion telle que définie ci-dessus.

L'invention permet de préparer des polymères aisément véhiculables étant donné que les dispersions ont des viscosités peu élevées, ce qui facilite leur mise en oeuvre dans les compositions cosmétiques.

Dans le domaine du maquillage, ces compositions apportent des propriétés de confort accrues, notamment un glissant amélioré, en particulier en environnement humide. De plus, les polymères ont une résistance et une tenue aux agressions externes, notamment aux frottements (repas) importante. Le confort et la tenue sont donc améliorés. Dans le cas des rouges à lèvres, l'utilisation de la salive sur les lèvres peut permettre de générer un gonflement, ou du volume, ce qui a un effet re-pulpeur; le film humide ainsi créé en surface protège le dépôt et donne une brillance 'mouillée'.
Dans le domaine capillaire, ces compositions peuvent conférer du volume et de la tenue à la chevelure. En outre, ces dispersions ou compositions peuvent permettent d'obtenir, en plus des effets coiffants et de la tenue, un toucher doux et non collant, de la douceur, ainsi qu'une facilité de démêlage et de lissage du cheveu, en milieu sec et/ou humide.
Un autre avantage de l'invention réside dans le fait que les particules de polymère peuvent être de très petite taille, notamment nanométrique, ce qui n'est pas le cas avec, par exemple, d'autres types de particules telles que des microsphères dont le diamètre est généralement supérieur à 1 micron. Or, une taille importante de l'ordre du micron a pour inconvénient d'entraîner une certaine visibilité des particules à l'oeil, lorsqu'elles sont dans une composition et lorsqu'elles sont appliquées sur la peau, ainsi qu'une mauvaise stabilité de la composition, notamment dans le temps.
Ainsi, les dispersions selon l'invention permettent l'obtention de compositions stables, qui peuvent en outre être transparentes, translucides ou opaques, au choix, selon la taille des particules de polymère qui y sont dispersées.
De plus, les dispersions selon l'invention sont dans des milieux huileux, particulièrement prisés dans le domaine du maquillage; or, les polymères comprenant des motifs PEG (polyéthylèneglycol) étaient jusqu'à présent généralement employés en solution ou dispersion aqueuse, donc plus difficilement formulables dans le domaine du maquillage.

Les dispersions selon l'invention sont donc constituées de particules, généralement sphériques, d'au moins un polymère éthylénique, stabilisé en surface par un stabilisant, dans un milieu non aqueux.
Les dispersions selon l'invention peuvent notamment se présenter sous forme de nanoparticules de polymères en dispersion stable dans un milieu non aqueux. Les nanoparticules sont de préférence d'une taille comprise entre 5 et 600 nm, notamment 10 à 500 nm, encore mieux 15 à 450 nm, étant donné qu'au-delà d'environ 600 nm, les dispersions de particules deviennent beaucoup moins stables.
Notamment, ces particules restent à l'état de particules élémentaires, sans former d'agglomérats, lorsqu'elles sont en dispersion dans lesdits milieux non aqueux.

Par polymère "éthylénique", on entend un polymère obtenu par polymérisation d'au moins 2 monomères, identiques ou différents, comprenant une insaturation éthylénique. Ledit polymère éthylénique peut être choisi par l'homme du métier en fonction de ses propriétés, selon l'application ultérieure souhaitée pour la composition. Ces polymères peuvent être en particulier réticulés.
Les polymères selon l'invention peuvent être des homopolymères ou des copolymères linéaires, branchés, greffés, ou même en étoiles. Ils peuvent être statistiques ou alternés. De préférence, ce sont des copolymères statistiques linéaires.

Le polymère éthylénique selon l'invention comprend 5% (inclus) à 50% (exclu) en poids de monomère hydrophile, ou d'un mélange de tels monomères, par rapport au poids total de monomères.
Il comprend de préférence de 7 à 49% en poids, mieux de 8 à 45% en poids, voire de 10 à 40% en poids, notamment

de 15 à 35% en poids, de monomère hydrophile, seul ou en mélange, par rapport au poids total de monomères initiaux.

Les monomères hydrophiles au sens de l'invention sont choisis parmi, seul ou en mélange, les monomères de formule (I) :

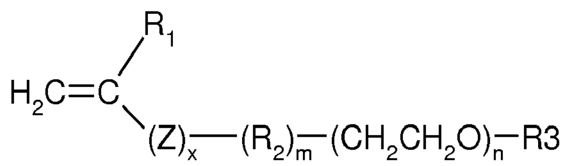

(I)

dans laquelle :

- R1 est un atome d'hydrogène ou un radical hydrocarboné, linéaire ou ramifié, de type $C_pH_{2p+1}$, avec p étant un entier compris entre 1 et 12 inclus;
- Z est un groupement divalent choisi parmi -COO-, -CONH-, $-CONCH_3-$, -OCO- , - O-, $-SO_2-$ -CO-O-CO- ou -CO-$CH_2$-CO-;
- x est 0 ou 1 ;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1 ;
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P;

et leurs sels.

Notamment, R1 peut représenter un radical méthyle, éthyle, propyle, butyle. De préférence, R1 représente l'hydrogène ou un radical méthyle.

De préférence, Z représente COO ou CONH, préférentiellement COO.

De préférence, x est égal à 1.

Dans le radical R2, le ou les hétéroatomes, quand ils sont présents, peuvent être intercalés dans la chaîne dudit radical R2, ou bien ledit radical R2 peut être substitué par un ou plusieurs groupes les comprenant tels que hydroxy, amino (NH2, NHR' ou NR'R" avec R' et R" identiques ou différents représentant un alkyle linéaire ou ramifié en C1-C22, notamment méthyle ou éthyle), -CF3, -CN, -SO3H ou - COOH.

En particulier, R2 peut comprendre un groupement -O-, -N(R)-, -CO- et leur combinaison, et notamment -O-CO-O-, -CO-O-, -N(R)CO-; -O-CO-NR-, -NR-CO-NR-, avec R représentant H ou un alkyle linéaire ou ramifié en C1-C22, comprenant éventuellement 1 à 12 hétéroatomes choisis parmi O, N, S, F, Cl, Br, Si et P.

Notamment R2 peut être :

- un radical alkylène ayant 1 à 20 atomes de carbone, tel que méthylène, éthylène, n-propylène, isopropylène, n-butylène, isobutylène, tertiobutylène, pentylène, iso-pentylène, n-hexylène, isohexylène, heptylène, isoheptylène, n-octylène, iso-octylène, nonylène, isononylène, décylène, isodécylène, n-dodécylène, isododécylène, tridécylène, n-tétradécylène, hexadécylène, n-octadécylène, docosanylène, arachinylène;
- un radical cycloalkylène ayant 5 à 10 atomes de carbone, substitué ou non, tel que cyclopentylène, cyclohexylène, cycloheptylène, cyclooctylne, cyclononylène, cyclo-décylène;
- un radical phénylène $-C_6H_4-$(ortho, méta ou para) éventuellement substitué, par un radical alkyle en C1-C12 comprenant éventuellement 1 à 18 hétéroatomes choisis parmi O, N, S, F, Si et P;
- un radical benzylène $-C_6H_4-CH_2-$ éventuellement substitué, par un radical alkyle en C1-C12 comprenant éventuellement 1 à 18 hétéroatomes choisis parmi O, N, S, F, Si et P;
- un radical de formule $-CH_2-O-CO-O-$, $CH_2-CH_2-O-CO-O-$, $-CH_2-CO-O-$, $-CH_2-CH_2-CO-O-$, $-CH_2-O-CO-NH-$, $-CH_2-CH_2-O-CO-NH-$; $-CH_2-NH-CO-NH-$, $-CH_2-CH_2-NH-CO-NH-$ ;$-CH_2-CHOH-$, $-CH_2-CH_2-CHOH-$, $-CH_2-CH_2-CH(NH_2)-$, $-CH_2-CH(NH_2)-$, - $CH_2-CH_2-CH(NHR')-$, $-CH_2-CH(NHR')-$, $-CH_2-CH_2-CH(NR'R")-$, $-CH_2-CH(NR'R")-$, - $CH_2-CH_2-CH_2-NR'-$, $-CH_2-CH_2-CH_2-O-$; $-CH_2-CH_2-CHR'-O-$ avec R' et R" représentant un alkyle linéaire ou ramifié en C1-C22 comprenant éventuellement 1 à 12 hétéroatomes choisis parmi O, N, S, F, Si et P;
- ou un mélange de ces radicaux.

De préférence R2 peut être :

- un radical alkylène ayant 1 à 20 atomes de carbone, notamment méthylène, éthylène, n-propylène, n-butylène, n-hexylène, n-octylène, n-dodécylène, n-octadécylène;
- un radical phénylène -$C_6H_4$-(ortho, méta ou para) éventuellement substitué, par un radical alkyle en C1-C12 comprenant éventuellement 1 à 18 hétéroatomes choisis parmi O, N, S, F, Si et P; ou
- un radical benzylène -$C_6H_4$-$CH_2$- éventuellement substitué par un radical alkyle en C1-C12 comprenant éventuellement 1 à 18 hétéroatomes choisis parmi O, N, S, F, Si et P.

De préférence, n est compris entre 5 et 200 inclus, et encore mieux entre 7 et 100 inclus, voire entre 9 et 50 inclus.

De préférence, R3 est un atome d'hydrogène; un radical phényle éventuellement substitué par un radical alkyle en C1-C12 comprenant éventuellement 1 à 20 hétéroatomes choisis parmi O, N, S, F, Si et P; un radical alkyle en C1-C30, notamment C1-C22, voire C2-C16, comprenant éventuellement 1 à 18 hétéroatomes choisis parmi O, N, S, F, Si et P; un radical cycloalkyle en C3-C12, notamment C4-C8, voire C5-C6, comprenant éventuellement 1 à 18 hétéroatomes choisis parmi O, N, S, F, Si et P.
Ces radicaux alkyle, cycloalkyle ou phényle peuvent comprendre notamment une ou plusieurs fonctions choisies parmi les fonctions suivantes:

| | | |
|---|---|---|
| Succinimido 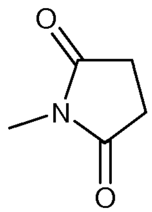 | Glutarate-succinimido 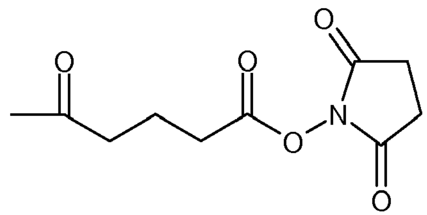 | Glutarate 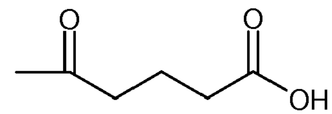 |
| maléimido 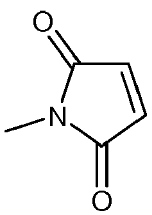 | Mésityle 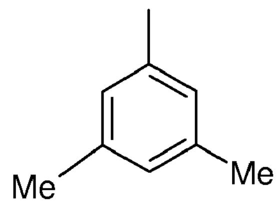 | Benzoate 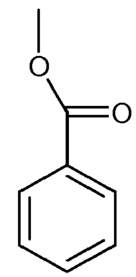 |
| Tosyle 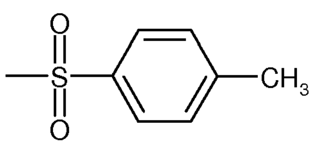 | Triéthoxysilane 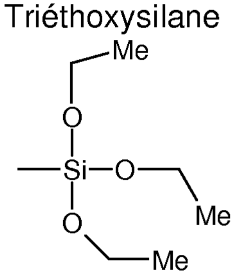 | Phtalimide 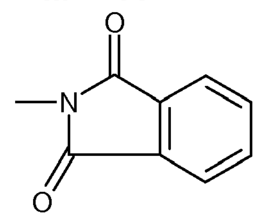 |
| Thioester 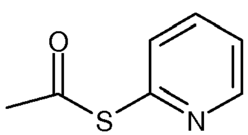 | Benzotriazole carbonate 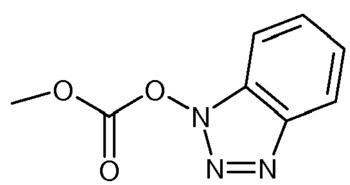 | Butyraldéhyde 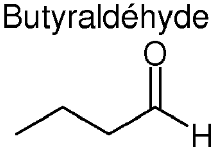 |

(suite)

| | |
|---|---|
| Acétaldéhyde diéthylacétal 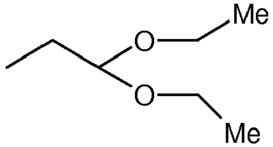 | Biotine 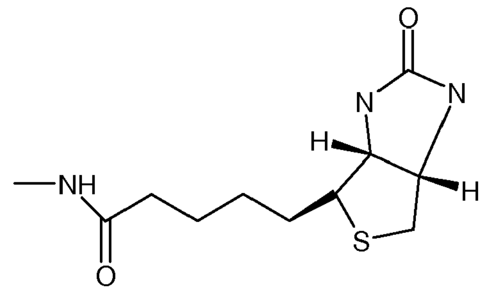 |
| Phospholipide 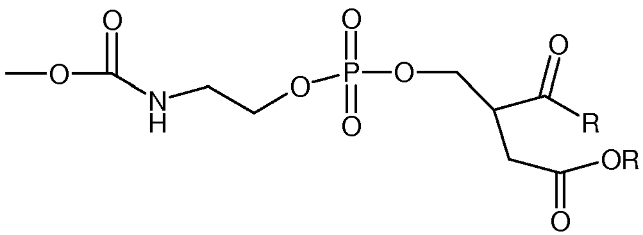 avec R = alkyle en C12-C18, et notamment lauryle, myristyle, palmityle, stéaryle, oléyle ou linoléyle | Succinate N-hydroxysuccinimide 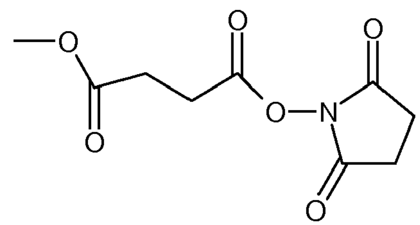 |

et/ou encore choisie parmi $-SO_3H$, -COOH, $-PO_4$, -NR5R6 ou $-N^+R5R6R7$, avec R5, R6 et R7, indépendamment l'un de l'autre, choisis parmi H ou alkyle en C1-C18, linéaire, ramifié ou cyclique, notamment méthyle, comprenant éventuellement 1 ou plusieurs hétéroatomes ou encore portant des groupements protecteurs tels que le t-butyloxycarbonyle (aussi appelé BOC) ou le 9-fluorenylmethoxycarbonyle (aussi appelé FmoC).

Parmi les radicaux R3, on peut citer les chaînes méthyle, éthyle, propyle, benzyle, éthylhexyle, lauryle, stéaryle, béhényle ($-(CH_2)_{21}-CH_3$), et également les chaînes alkyles fluorées telles que par exemple heptadecafluorooctyl sulfonyl amino éthyle $CF_3-(CF_2)_7-SO_2-N(C_2H_5)-CH_2-CH_2$; ou encore les chaînes $-CH_2-CH_2-CN$, succinimido, maléimido, mésityle, tosyle, triéthoxysilane ou phtalimide.

Préférentiellement, les monomères de formule (I) sont tels que :

- R1 = H ou méthyle,
- Z représente COO,
- x=1
- m=0,
- n= 7 à 100 inclus,
- R3 est choisi parmi un atome d'hydrogène; un radical phényle éventuellement substitué par un radical alkyle en C1-C12; un radical alkyle en C1-C30, notamment C1-C22, voire C2-C16.

Parmi les monomères de formule (I) particulièrement préférés, on peut citer :

- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle, Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle.

Des exemples de monomères commerciaux sont :

- le CD 350 (méthacrylate de méthoxy-poly(éthylène glycol 350) et le CD 550 (méthacrylate de méthoxy-poly(éthylène glycol 550), fourni par SARTOMER Chemicals;
- le M90G (méthacrylate de méthoxy-poly(éthylène glycol (9 unités de répétition)) et le M230G (méthacrylate de méthoxy-polyéthylène glycol (23 unités de répétitions)) disponibles chez Shin-Nakamura Chemicals;
- les méthacrylates de méthoxy-poly(éthylène glycol) de poids moléculaires moyens 300, 475 ou 1100, disponibles chez Sigma-Aldrich;
- l'acrylate de méthoxy-poly(éthylène glycol) de poids moléculaire moyen 426 disponible chez Sigma-Aldrich;
- les méthacrylates de méthoxy-poly(éthylène glycol) disponibles chez LAPORTE sous les dénominations commerciales : MPEG 350, MPEG 550, S10W, S20W, ou chez Cognis sous la dénomination BISOMER.
- les poly(éthylène glycol) monomethyl éther, mono(succinimidyl succinate) ester de poids moléculaire moyen 1900 ou 5000, de chez Polysciences;
- le méthacrylate de béhényl poly(éthylène-glycol PEG-25), disponible chez Rhodia, sous la dénomination SIPOMER BEM;
- les acrylates de poly(éthylène glycol) phényl éther de poids moléculaires moyens 236, 280 ou 324 disponibles chez Aldrich;
- le méthoxy polyéthylène glycol 5000 2-(vinyl sulfonyl) éthyl éther disponible commercialement chez Fluka;
- le méthacrylate de polyéthylène glycol éthyl éther disponible chez Aldrich ;
- les méthacrylates de polyéthylène glycol 8000, 4000, 2000 de Monomer & Polymer Dajac laboratories.

Les monomères de formule (I) tout particulièrement préférés sont choisis parmi les (méth)acrylates de poly (éthylène glycol) et les (méth)acrylates d'alkyl-poly(éthylène glycol), plus particulièrement les méthacrylates de méthyl-poly(éthylène glycol),

Le polymère éthylénique selon l'invention peut bien évidemment comprendre un seul monomère de formule (I) ou un mélange de tels monomères.

Les polymères présents dans la dispersion sont donc issus de la polymérisation d'un ou plusieurs monomères de formule (I) et d'un ou plusieurs monomères additionnels, qui sont donc présents à raison de 50 (inclus) à 95% en poids, notamment de 51 à 97% en poids, voire de 55% à 98% en poids, et encore mieux de 60% à 90% en poids, préférentiellement de 65 à 85% en poids, par rapport au poids total de monomères.

Parmi les monomères additionnels susceptibles d'être employés dans le cadre de la présente invention, on

peut citer, seul ou en mélange, les monomères de formule (II), et leurs sels:

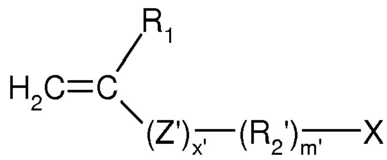

(II)

dans lesquelles :

- R1 est un atome d'hydrogène ou un radical hydrocarboné, linéaire ou ramifié, de type $C_pH_{2p+1}$, avec p étant un entier compris entre 1 et 12, inclus; Notamment, R1 peut représenter un radical méthyle, éthyle, propyle, butyle. De préférence, R1 représente l'hydrogène ou un radical méthyle.

- Z' est un groupement divalent choisi parmi -COO- et -CONH-,

- x' est 0 ou 1, de préférence 1.

- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P; Notamment R'2 peut être :

- un radical alkylène tel que méthylène, éthylène, propylène, n-butylène, isobutylène, tertiobutylène, n-hexylène, n-octylène, n-dodécylène, n-octadécylène, n-tétradécylène, n-docosanylène;
- un radical phénylène $-C_6H_4-$(ortho, méta ou para) éventuellement substitué, par un radical alkyle en C1-C12 comprenant éventuellement 1 à 18 hétéroatomes choisis parmi N, O, S, F, Si et/ou P; ou bien un radical benzylène $-C_6H_4-CH_2-$ éventuellement substitué, par un radical alkyle en C1-C12 comprenant éventuellement 1 à 18 hétéroatomes choisis parmi O, N, S, F, Si et P;
- ou un mélange de ces radicaux;
- m' est 0 ou 1 ;
- X est choisi parmi -H, -COOH, $-SO_3H$, $-OSO_3H$, $-PO_3H_2$ et $-OPO_3H_2$.

Parmi les monomères de formule (II) préférés, on peut citer, seul ou en mélange, le (méth)acrylamide, l'acide (méth)acrylique, l'acide crotonique, l'acide itaconique, l'acide fumarique, l'acide maléique, l'acide diacrylique, l'acide diméthylfumarique, l'acide citraconique, l'acide acrylamidopropanesulfonique, l'acide 2-acrylamido 2-méthylpropanesulfonique, l'acide styrènesulfonique, l'acide vinylbenzoïque, l'acide vinylphosphorique, l'acide vinylsulfonique, l'acide vinylbenzène sulfonique, l'acide acrylamidoglycolique de formule CH2=CH-CONHCH(OH)COOH, l'acide vinylphosphonique; le (méth)acrylate de sulfopropyle ($CH_2=C(CH_3)CO_2(CH_2)_3SO_3H$), le (méth)acrylate de sulfoéthyle, la vinylméthylsulfone, le 2-(méthacryloyloxy)éthylphosphate de formule $CH_2=C(CH_3)COOC_2H_4OP(O)(OH)_2$; ainsi que leurs sels; et leurs mélanges.

Les monomères additionnels peuvent également être choisis parmi les monomères suivants, seuls ou en mélange :

- (i) les esters de l'acide (méth)acrylique de formule $CH_2=CHCOOR4$ ou $CH_2=C(CH_3)COOR4$ avec R4 représentant une chaîne carbonée, notamment hydrocarbonée (alkyle), ayant 1 à 32 atomes de carbone, linéaire, cyclique ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F).
  En particulier, la chaîne carbonée R4 peut être :

  - un groupe alkyle en C1-C32;
  - un groupe cycloalkyle en C3 à C8;
  - un groupe aryle en C6 à C20 ;
  - un groupe aralkyle en C7 à C30 (groupe alkyle en C1 à C4);
  - un groupe hétérocyclique de 4 à 12 chaînons contenant un ou plusieurs hétéroatomes choisis parmi O, N et S;
  - un groupe hétérocycloalkyle (alkyl de C1 à C4) tel qu'un groupe furfuryle;

lesdits groupes alkyle, cycloalkyle, aryle, aralkyle, hétérocyclique ou hétérocycloalkyle pouvant comprendre intercalé(s) un ou plusieurs hétéroatomes choisis parmi O, N, S et/ou être substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, et/ou lesdits groupes alkyle pouvant, en outre, être éventuellement substitués par un ou plusieurs substituants choisis parmi le groupe hydroxyle et les atomes d'halogène (Cl, Br, I et F).
On peut ainsi citer les (méth)acrylates de méthyle, d'éthyle, de propyle, de n-butyle, d'isobutyle, de 2-éthylhexyle, d'octyle, d'isooctyle, d'isodécyle, de décyle, de lauryle, de tridécyle, de dodécyle, de myristyle, de cétyle, de palmityle, de stéaryle, de béhényle, d'oléyle; de tertiobutyle, de t-butylcyclohexyle, de t-butylbenzyle, de furfuryle et d'isobornyle; les (méth)acrylates de 2-hydroxyéthyle, d'éthoxyéthyle, de 2-méthoxyéthyle et d'hydroxypropyle.

- (ii) les (méth)acrylamides de formule $CH_2$=CHCONR5R'5 ou $CH_2$=C($CH_3$)CONR5R'5 dans lesquelles R5 et R'5, identiques ou différents, représentent un atome d'hydrogène ou une chaîne carbonée, notamment hydrocarbonée (alkyle), ayant 7 à 28 atomes de carbone, linéaire, cyclique ou ramifiée, saturée ou insaturée, éventuellement aromatique, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F). En particulier, la chaîne carbonée R5 et/ou R'5 peut être :

  - un groupe alkyle en C7-C28;
  - un groupe cycloalkyle en C7 à C8;
  - un groupe aryle en C7 à C20 ;
  - un groupe aralkyle en C7 à C28 (groupe alkyle en C1 à C4);
  - un groupe hétérocyclique de 4 à 12 chaînons contenant un ou plusieurs hétéroatomes choisis parmi O, N et S;
  - un groupe hétérocycloalkyle (alkyl de C1 à C4) tel qu'un groupe furfuryle;

  lesdits groupes alkyle, cycloalkyle, aryle, aralkyle, hétérocyclique ou hétérocycloalkyle pouvant comprendre intercalé(s) un ou plusieurs hétéroatomes choisis parmi O, N, S et/ou être substitué par un ou plusieurs substituants choisis parmi le groupe hydroxyle, les atomes d'halogène et les groupes alkyles en C1-C4, linéaires ou ramifiés dans lesquels se trouve(nt) éventuellement intercalé(s) un ou plusieurs hétéroatomes choisis parmi O, N, S et P, et/ou lesdits groupes alkyle pouvant, en outre, être éventuellement substitués par un ou plusieurs substituants choisis parmi le groupe hydroxyle et les atomes d'halogène (Cl, Br, I et F).
  Des exemples de tels monomères sont la N-tertbutyl(méth)acrylamide, la N-butyl(méth)acrylamide, la N-isobutyl(méth)acrylamide, la N-propylméthacrylamide, la N-isopropylméthacrylamide, la N-hexyl(méth)acrylamide, la N-2-éthylhexyle(méth)acrylamide, la N-octyl(méth)acrylamide, la N-isooctyl(méth)acrylamide, la N-nonyl(méth)acrylamide, la N-undécyl(méth)acrylamide, la N-dodécyle(méth)acrylamide, la N-tridécyle(méth)acrylamide, la N-tétradécyle(méth)acrylamide, la N-hexadécyle(méth)acrylamide, la N-palmityle(méth)acrylamide, la N-octadécyle(méth)acrylamide, la N-docosanoyle(méth)acrylamide, la N-octadécénoyle(méth)acrylamide, la N-cyclohexyle(méth)acrylamide, la N-phényle(méth)acrylamide, la N-isobornyle(méth)acrylamide, la N-benzyle(méth)acrylamide, la N,N-dibutyl (méth)acrylamide; la N,N-diéthylaminopropyl(meth)acrylamide.

- (iii) les esters vinyliques de formule $CH_2$=CH-OCO-R6 avec R6 représentant une chaîne carbonée, notamment hydrocarbonée, ayant 1 à 12 atomes de carbone, linéaire ou ramifiée, saturée ou insaturée, parmi lesquels on peut citer l'acétate de vinyle, le butyrate (ou butanoate) de vinyle, l'éthylhexanoate de vinyle, le néononanoate de vinyle, le néododécanoate de vinyle, le propionate de vinyle, l'hexanoate de vinyle, l'éthylhexanoate de vinyle, l'octanoate de vinyle, le décanoate de vinyle, le pivalate de vinyle, le palmitate de vinyle, le stéarate de vinyle, le cyclohexanoate de vinyle, le benzoate de vinyle, le 4-tert-butylbenzoate de vinyle;

- (iv) les éthers de vinyle de formule $CH_2$=CHOR7 avec R7 représentant une chaîne carbonée, notamment hydrocarbonée, ayant 2 à 12 atomes de carbone, linéaire ou ramifiée, saturée ou insaturée; parmi lesquels on peut citer l'éthylvinyléther, l'éthylhexylvinyléther, le butylvinyléther; l'isobutylvinyléther, le cyclohexylvinyléther, l'octylvinyléther, le décylvinyléther, le dodécylvinyléther, l'hexadécylvinyléther et l'octadécylvinyléther.

- (v) les composés vinyliques de formule $CH_2$=CHR8 dans laquelle R8 est

  - un groupe hydroxyle;
  - un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes

d'halogène (Cl, Br, I et F);

- un groupe cycloalkyle en $C_3$ à $C_8$ tel que cyclohexane,
- un groupe aryle en $C_6$ à $C_{20}$ tel que phényle,
- un groupe aralkyle en $C_7$ à $C_{30}$ (groupe alkyle en $C_1$ à $C_4$) tel que 2-phényléthyle ou benzyle,
- un groupe hétérocyclique de 4 à 12 chaînons contenant un ou plusieurs hétéroatomes choisis parmi O, N, et S,
- un groupe hétérocycloalkyle (alkyle de 1 à 4 carbones), tel que furfuryle, furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I et F).

Des exemples de monomères vinyliques sont le vinylcyclohexane, le styrène, le vinylcaprolactame, le méthylstyrène; le 4-tert-butylstyrene, le 4-acétoxystyrene; le 4-méthoxystyrene, le 3-méthylstyrene; le 4-méthylstyrene, le 2-chlorostyrene, le 3-chlorostyrene, le 4-chlorostyrene, le dimethylstyrene; le 2,6-dichlorostyrene; le 2,4-dimethylstyrene; le 2,5-dimethylstyrene; le 3,5-éthoxystyrene; le 2,4-éthoxystyrene, le vinylbutyral; le chlorure de vinyle; le vinylformal; le vinylidène chlorure, le vinylidène fluorure, le 2-vinylnaphtalène; le N-méthyl maléimide; le 1-octene, le 1-butene; le chlorobutadiène, le chlorotrifluoroethylene; le cis-isoprene, le trans-isoprene; le 1-octadecene; le butadiène; l'hexadécène, l'eicosène, le 4-fluorostyrene.

Parmi les monomères additionnels tout particulièrement préférés, on peut citer les monomères suivants, et leurs mélanges :

- les esters de l'acide (méth)acrylique avec R4 en C1-C18, et notamment les (méth)acrylates de méthyle, d'éthyle, de propyle, de n-butyle, d'isobutyle, de 2-éthylhexyle, de tertiobutyle, d'isooctyle, de décyle, de myristyle, de stéaryle, d'isobornyle,
- le styrène, l'éthylhexylvinylether, le dodécylvinyléther, l'hexanoate de vinyle;
- l'acide acrylique et l'acide méthacrylique.

Lorsqu'il comporte des fonctions acides, le polymère selon l'invention peut être neutralisé, totalement ou partiellement, par une base organique, par exemple une amine ou une alkylamine, primaire, secondaire ou tertiaire, l'amine pouvant comporter ou non des substituants (hydroxyle) et/ou un ou plusieurs atomes d'azote et/ou d'oxygène. On peut notamment citer l'amino-2-méthyl-2-propanol, la triéthylamine, la butylamine, la triéthanolamine, la diméthylamino-2-propanol, la lysine, la 3-(diméthylamino)propylamine, la cyclohexylamine, la N-octylamine, la N-dodécylamine et la N-octadécylamine.

Lorsqu'il comporte des fonctions basiques, le polymère selon l'invention peut être neutralisé, totalement ou partiellement, par des acides organiques qui peuvent comporter un ou plusieurs groupes carboxylique, sulfonique, ou phosphonique. Il peut s'agir d'acides aliphatiques linéaires, ramifiés ou cycliques ou encore d'acides aromatiques. On peut notamment citer l'acide acétique, l'acide alphahydroxyéthanoïque, l'acide alpha-hydroxyoctanoïque, l'acide alphahydroxycaprylique, l'acide ascorbique, l'acide benzoïque, l'acide béhénique, l'acide caprique, l'acide caproïque, l'acide caprylique, l'acide citrique, l'acide dodécylbenzène sulfonique, l'acide 2-éthylcaproïque, l'acide folique, l'acide fumarique, l'acide galactarique, l'acide gluconique, l'acide glycolique, l'acide 2-hexadécyl eicosanoïque, l'acide hydroxycaproique, l'acide 12-hydroxystéarique, l'acide isolaurique (ou 2-butyl octanoïque), l'acide isomyristique (ou 2-hexyl octanoïque), l'acide isoarachidique (ou 2-octyl dodécanoïque), l'acide isolignocérique (ou 2-décyl tétradécanoïque), l'acide lactique, l'acide laurique, l'acide malique, l'acide myristique, l'acide oléïque, l'acide palmitique, l'acide propionique, l'acide sébacique, l'acide stéarique, l'acide tartrique, l'acide téréphtalique, l'acide trimésique, l'acide undécylénique, la propylebétaïne, la cocoamidopropylbétaïne, et leurs mélanges.

Il est possible de neutraliser les monomères possédant une fonction acide et/ou amine neutralisable avant leur polymérisation, ou bien de neutraliser le polymère une fois formé. De préférence, on neutralise le polymère après sa formation.

De préférence, le polymère ne présente qu'une température de transition vitreuse. Toutefois, il peut présenter plusieurs températures de transition vitreuse, notamment deux Tg; dans ce cas, de préférence, la Tg la plus basse est inférieure à +20°C.

Les polymères utilisables dans le cadre de la présente invention ont de préférence un poids moléculaire moyen en nombre (Mn) compris entre 2000 et 1 000 0000, notamment entre 3000 et 800 000, et encore mieux entre 4000 et 500 000, voire entre 10 000 et 300 000.

La dispersion de particules de polymères selon l'invention comprend donc un milieu non aqueux, dans lequel sont dispersées lesdites particules.

Ce milieu non aqueux est constitué d'au moins un composé non aqueux, liquide à 25°C, ayant un paramètre de solubilité global selon l'espace de solubilité de HANSEN inférieur ou égal à 20 $(MPa)^{1/2}$, ou un mélange de tels composés.

Le paramètre de solubilité global δ selon l'espace de solubilité de HANSEN est défini dans l'article "Solubility parameter values" de Grulke, dans l'ouvrage "Polymer Handbook" 3ème édition, Chapitre VII, pages 519-559 par la relation :

$$\delta = ( d_D^2 + d_P^2 + d_H^2)^{1/2}$$

dans laquelle :

- $d_D$ caractérise les forces de dispersion de LONDON issues de la formation de dipôles induits lors des chocs moléculaires,
- $d_P$ caractérise les forces d'interactions de DEBYE entre dipôles permanents,
- $d_H$ caractérise les forces d'interactions spécifiques (type liaisons hydrogène, acide/base, donneur/accepteur, etc.).

La définition des solvants dans l'espace de solubilité tridimensionnel selon HANSEN est décrite dans l'article de HANSEN : "The three dimensionnal solubility parameters" J. Paint Technol. 39, 105 (1967).

Parmi les composés liquides non aqueux ayant un paramètre de solubilité global selon l'espace de solubilité de HANSEN inférieur ou égal à 20 $(MPa)^{1/2}$, on peut citer les corps gras liquides, notamment les huiles, qui peuvent être choisies parmi les huiles naturelles ou synthétiques, carbonées, hydrocarbonées, fluorées et/ou siliconées, éventuellement ramifiées, seules ou en mélange.

Parmi ces huiles, on peut citer les huiles végétales formées par des esters d'acides gras et de polyols, en particulier les triglycérides, telles que l'huile de tournesol, de sésame ou de colza, ou les esters dérivés d'acides ou d'alcools à longue chaîne (c'est à dire ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle.

On peut également citer les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.

On peut encore citer les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.

On peut également citer les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.

Parmi les composés non aqueux susceptibles d'être employés, on peut également citer les monoalcools ayant un paramètre de solubilité global selon l'espace de solubilité de HANSEN inférieur ou égal à 20 $(MPa)^{1/2}$, c'est-à-dire les monoalcools gras aliphatiques ayant au moins 6 atomes de carbone, notamment 6 à 32, la chaîne hydrocarbonée ne comportant pas de groupement de substitution. Comme monoalcools selon l'invention, on peut citer l'alcool oléique, le décanol, le dodécanol, l'octadécanol et l'alcool linoléique.

Dans un mode préféré, le milieu non aqueux comprend des huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle; ainsi que leurs mélanges.

Dans un autre mode préféré, le milieu non aqueux comprend des hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.

Le choix du milieu non aqueux peut être effectué aisément par l'homme du métier en fonction de la nature des monomères constituant le polymère et/ou de la nature du stabilisant.

La dispersion de polymère peut être fabriquée comme décrit dans le document EP-A-749747. D'une manière

générale, la polymérisation peut être effectuée en dispersion, c'est-à-dire par précipitation du polymère au cours de formation, avec protection des particules formées à l'aide d'un stabilisant.

On peut donc préparer un mélange comprenant les monomères initiaux ainsi qu'un amorceur radicalaire. Ce mélange est dissous dans un solvant de synthèse.

Les monomères sont solubles dans le milieu réactionnel, alors que le polymère n'y est pas soluble. Au fur et à mesure de la polymérisation, le polymère va précipiter et se trouver stabilisé par le stabilisant présent. On obtient ainsi des particules de polymères protégées en surface par le stabilisant.

On peut directement effectuer la polymérisation dans le milieu non aqueux qui peut donc jouer également le rôle de solvant de synthèse.

On peut également effectuer la polymérisation dans un solvant de synthèse, puis effectuer ensuite un échange de solvant, en remplaçant le solvant de synthèse par le milieu non aqueux.

Ainsi, lorsque le milieu non aqueux choisi est une huile non volatile, hydrocarbonée ou siliconée, on peut effectuer la polymérisation dans un solvant organique apolaire (solvant de synthèse) puis ajouter l'huile non volatile (qui doit être miscible avec ledit solvant de synthèse) et distiller sélectivement le solvant de synthèse.

On choisit donc de préférence un solvant de synthèse tel que les monomères initiaux, et l'amorceur radicalaire, y sont solubles, et les particules de polymère obtenu y sont insolubles afin qu'elles y précipitent lors de leur formation. En particulier, on peut choisir le solvant de synthèse parmi les alcanes tels que l'heptane, l'isododécane ou le cyclohexane.

Lorsque le milieu non aqueux choisi est une huile volatile, hydrocarbonée ou siliconée, on peut directement effectuer la polymérisation dans ladite huile qui joue donc également le rôle de solvant de synthèse. Les monomères doivent de préférence également y être solubles, ainsi que l'amorceur radicalaire, et le polymère obtenu doit y est insoluble.

Les monomères sont de préférence présents dans le solvant de synthèse, avant polymérisation, à raison de 5-80% en poids. La totalité des monomères peut être présente dans le solvant avant le début de la réaction, ou une partie des monomères peut être ajoutée au fur et à mesure de l'évolution de la réaction de polymérisation.

L'amorceur radicalaire peut être, par exemple, un composé azoïque ou peroxyde tels que l'azo-bis-isobutyronitrile ou le tertiobutylperoxy-2-éthyl hexanoate.

Les particules de polymère sont stabilisées en surface.

Dans un premier mode de réalisation, les particules peuvent être stabilisées en surface au fur et à mesure de la polymérisation, grâce à un stabilisant qui peut être notamment un polymère séquencé, un polymère greffé, et/ou un polymère statistique, seul ou en mélange. La stabilisation peut être effectuée par tout moyen connu, et en particulier par polymérisation en présence du stabilisant.

De préférence, le stabilisant est présent dans le mélange au départ de la polymérisation. Toutefois, il est également possible de l'ajouter en continu, notamment lorsqu'on ajoute également des monomères en continu.

Dans un second mode de réalisation, le polymère peut être synthétisé dans un solvant de synthèse, puis mis en dispersion dans un milieu non aqueux de dispersion par ajout du dispersant, et le solvant de synthèse évaporé.

On peut utiliser 0,1 à 30% en poids de stabilisant par rapport au poids du mélange initial de monomères, et de préférence de 1 à 25% en poids, préférentiellement de 2 à 20% en poids, voire de 3 à 15% en poids.

Lorsqu'on utilise un polymère greffé et/ou séquencé en tant que stabilisant, on choisit le solvant de synthèse de telle manière qu'au moins une partie des greffons ou séquences dudit polymère-stabilisant soit soluble dans ledit solvant, l'autre partie des greffons ou séquences n'y étant pas soluble. Le polymère-stabilisant utilisé lors de la polymérisation doit être soluble, ou dispersible, dans le solvant de synthèse. De plus, on choisit de préférence un stabilisant comportant une partie (séquences, greffons ou autre), présentant une certaine affinité pour le polymère formé lors de la polymérisation.

Lorsqu'on utilise un polymère statistique en tant que stabilisant, on le choisit de manière à ce qu'il possède une quantité suffisante de groupements le rendant soluble dans le solvant de synthèse envisagé.

Parmi les polymères greffés, on peut citer les polymères siliconés greffés avec une chaîne hydrocarbonée et les polymères hydrocarbonés greffés avec une chaîne siliconée.

Conviennent également les copolymères greffés ayant par exemple un squelette insoluble de type polyacrylique avec des greffons solubles de type acide poly (12-hydroxy stéarique).

On peut également citer, comme polymère stabilisant :

- (a) les copolymères blocs greffés ou séquencés comprenant au moins un bloc de type polyorganosiloxane et au moins un bloc d'un polymère (i) issu de la polymérisation radicalaire ou (ii) issu d'une polycondensation, notamment de type polyéther, polyester ou polyamide, ou leur mélange, ledit copolymère pouvant comporter des entités fluorées;

  Comme copolymères blocs greffés ou séquencés comprenant au moins un bloc de type polyorganosiloxane et au moins un bloc d'un polymère radicalaire, on peut citer les copolymères greffés de type acrylique/silicone qui peuvent être employés notamment lorsque le milieu non aqueux est siliconé.

  Lorsque les copolymères blocs greffés ou séquencés comprennent au moins un bloc de type polyorganosiloxane

et au moins un bloc polyéther, le bloc polyorgano-polysiloxane peut être notamment un polydiméthylsiloxane ou bien encore un polyalkyl($C_2$-$C_{18}$)méthylsiloxane; le bloc polyéther peut être un polyalkylène en $C_2$-$C_{18}$, en particulier polyoxyéthylène et/ou polyoxypropylène. On peut ainsi utiliser les diméthicones copolyol ou encore les alkyl($C_2$-$C_{18}$) méthicones copolyol, éventuellement réticulés. On peut par exemple utiliser le diméthicone copolyol vendu sous la dénomination "DOW CORNING 3225C" par la société DOW CORNING, ou le lauryl méthicone copolyol vendu sous la dénomination "DOW CORNING Q2-5200 par la société "DOW CORNING".
On peut aussi citer le lauryl diméthicone copolyol crosspolymer, par exemple le KSG31 ou KSG32 de Shin-Etsu, le cétyl diméthicone copolyol tel que le DMC 3071 de GE, et le diméthicone copolyol PPG-3-oléyl éther tel que le KF-6026 de Shin Etsu.

- (b) les copolymères blocs, greffés ou séquencés, de (méth)acrylates d'alkyle en C1-C4 et de (méth)acrylates d'alkyle en C8-C30. On peut citer le copolymère méthacrylate de stéaryle/méthacrylate de méthyle.

- (c) les copolymères blocs greffés ou séquencés comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d'un polyéther, d'un polyester ou d'un polyamide, ou leurs mélanges.
Notamment, on peut utiliser les copolymères comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique. Lorsque le monomère éthylénique comporte plusieurs liaisons éthyléniques éventuellement conjuguées, les insaturations éthyléniques résiduelles après la polymérisation sont généralement hydrogénées. Ainsi, de façon connue, la polymérisation de l'isoprène conduit, après hydrogénation, à la formation de bloc éthylène-propylène, et la polymérisation de butadiène conduit, après hydrogénation, à la formation de bloc éthylène-butylène. Parmi ces copolymères séquencés, on peut citer les copolymères de type "dibloc" ou "tribloc" du type polystyrène/polyisoprène, polystyrène/polybutadiène tels que ceux vendus sous le nom de 'LUVITOL HSB' par BASF, du type polystyrène/copoly(éthylène-propylène) tels que ceux vendus sous le nom de 'KRATON' par Shell Chemical Co ou encore du type polystyrène/copoly(éthylène-butylène).

Comme copolymères blocs greffés ou séquencés comprenant au moins un bloc résultant de la polymérisation d'au moins un monomère éthylénique, tels que l'éthylène, l'isobutylène, et d'au moins un bloc d'un polymère acrylique tel que le méthacrylate de méthyle, on peut citer les copolymères bi- ou triséquencés poly(méthacrylate de méthyle)/polyisobutylène ou les copolymères greffés à squelette poly(méthacrylate de méthyle) et à greffons polyisobutylène.

Comme copolymères blocs greffés ou séquencés comprenant au moins un bloc résultant de la polymérisation d'au moins un monomère éthylénique et d'au moins un bloc d'un polyéther tel qu'un polyoxyalkylène en C2-C18, en particulier polyoxyéthylène et/ou polyoxypropylène, on peut citer les copolymères bi- ou triséquencés polyoxyéthylène/polybutadiène ou polyoxyéthylène/polyisobutylène.

On peut également utiliser comme stabilisant, des composés tels que :

- (d) les alkyl-dimethicones dans lesquels le groupement alkyl comprend 6 à 32 atomes de carbone, tels que le lauryle méthicone et le stéaryle méthicone, notamment Si tec LDM 3107 d'ISP, le cétyl diméthicone tel que l'ABIL Wax 9801, le behenoxydimethicone tel que l'ABIL 5440 de Goldschmidt

- (e) les dimethiconol ester de formule :

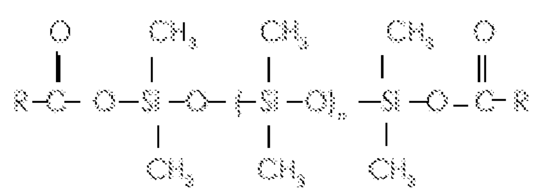

dans laquelle R est un radical alkyle ayant 6 à 32 atomes de carbone, tel que le dimethiconol béhénate, et notamment les produits ULTRABEE de NOVEON et Pecosil DB de Phoenix Chemical.

- (f) les alkylamidoamines ayant notamment 6 à 60 atomes de carbone, notamment 12 à 50, telles que la behenamidopropyldimethylamine et notamment le Catemol 220 de Phoenix Chemical, de formule :

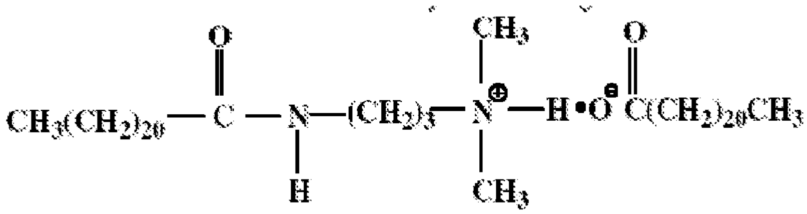

- (g) les copolymères comprenant au moins une partie polyorganosiloxane et des groupements fluorés, et notamment les silicones fluorées ou fluorosilicones qui peuvent être représentées par la formule :

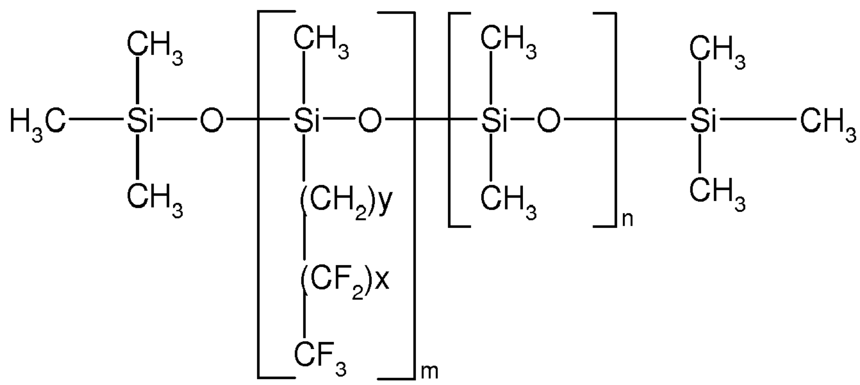

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000 et 15000; et plus particulièrement les perfluorononyldimethicone, tels que ceux vendus sous la dénomination PECOSIL FSH-150 et 300 ou PECOSIL FSL-150 et 300 par Phoenix Chemical;

Lorsque le solvant de synthèse est apolaire, il est préférable de choisir en tant que stabilisant, un polymère apportant une couverture des particules la plus complète possible, plusieurs chaînes de polymères-stabilisants venant alors s'adsorber sur une particule de polymère obtenu par polymérisation.

Dans ce cas, on préfère alors utiliser comme stabilisant, soit un polymère greffé, soit un polymère séquencé, de manière à avoir une meilleure activité interfaciale. En effet, les séquences ou greffons insolubles dans le solvant de synthèse apportent une couverture plus volumineuse à la surface des particules.

Lorsque le solvant de synthèse liquide comprend au moins une huile de silicone, l'agent stabilisant est de préférence choisi dans le groupe constitué par les copolymères blocs greffés ou séquencés comprenant au moins un bloc de type polyorganosiloxane et au moins un bloc d'un polymère radicalaire ou d'un polyéther ou d'un polyester comme les blocs polyoxypropyléné et/ou oxyéthyléné.

Lorsque le solvant de synthèse liquide comprend au moins une huile carbonée, notamment de type hydrocarbure, plus particulièrement isoparaffine, l'agent stabilisant est de préférence choisi parmi les copolymères blocs greffés ou séquencés comprenant au moins un bloc résultant de la polymérisation d'au moins un monomère éthylénique, à une ou plusieurs liaisons éthyléniques éventuellement conjuguées, tels que l'éthylène, le butadiène, l'isoprène, et d'au moins un bloc d'un polymère styrénique; et plus particulièrement l'agent stabilisant est un copolymère "dibloc" ou "tribloc" du type polystyrène/polyisoprène, polystyrène/polybutadiène, polystyrène/copoly(éthylène-propylène), polystyrène/copoly (éthylène-butylène).

Il est possible d'ajouter à la dispersion de polymères, un plastifiant de manière à abaisser la Tg des polymères utilisés. Le plastifiant peut être choisi parmi les plastifiants usuellement utilisés dans le domaine d'application et notamment parmi les composés susceptibles d'être des solvants du polymère. Le plastifiant peut être intégré lors de la synthèse ou ajouté une fois la synthèse réalisée.

Les dispersion obtenues selon l'invention peuvent alors être utilisées dans une composition notamment cosmétique ou pharmaceutique, qui comprend par ailleurs un milieu cosmétiquement ou pharmaceutiquement acceptable. Selon l'application envisagée, on peut utiliser des dispersion de polymères filmifiables ou non filmifiables, dans un milieu non aqueux comprenant des huiles volatiles ou non volatiles.

La composition peut alors comprendre, selon l'application envisagée, les constituants habituels à ce type de composition.

Parmi ces constituants, on peut citer les cires, les huiles, les gommes et/ou les corps gras pâteux, d'origine végétale, animale, minérale ou de synthèse, voire siliconés, et leurs mélanges.

Parmi les cires susceptibles d'être présentes dans la composition selon l'invention, on peut citer, seules ou en mélange, les cires hydrocarbonées telles que la cire d'abeilles; la cire de Carnauba, de Candellila, d'Ouricoury, du Japon, les cires de fibres de lièges ou de canne à sucre; les cires de paraffine, de lignite; les cires microcristallines; la cire de lanoline; la cire de Montan; les ozokérites; les cires de polyéthylène; les cires obtenues par synthèse de Fischer-Tropsch; les huiles hydrogénées, les esters gras et les glycérides concrets à 25°C. On peut également utiliser des cires de silicone, parmi lesquelles on peut citer les alkyls, alcoxys et/ou esters de polyméthylsiloxane.

La composition selon l'invention peut également comprendre des huiles d'origine minérale, animale, végétale ou synthétique, carbonées, hydrocarbonées, fluorées et/ou siliconées, seules ou en mélange dans la mesure où elles forment un mélange homogène et stable et où elles sont compatibles avec l'utilisation envisagée. Parmi les huiles susceptibles d'être présentes dans la composition selon l'invention, on peut citer, seules ou en mélange, les huiles hydrocarbonées telles que l'huile de paraffine ou de vaseline; le perhydrosqualène; l'huile d'arara; l'huile d'amande douce, de calophyllum, de palme, de ricin, d'avocat, de jojoba, d'olive ou de germes de céréales; des esters d'acide lanolique, d'acide oléique, d'acide laurique, d'acide stéarique; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones. On peut également utiliser des huiles volatiles, telles que la cyclotétradiméthylsiloxane, la cyclopentadiméthylsiloxane, la cyclohexadiméthylsiloxane, le méthylhexyldiméthylsiloxane, l'hexaméthyldisiloxane ou les isoparaffines.

La composition selon l'invention peut également comprendre une ou plusieurs matières colorantes choisies parmi les composés pulvérulents et/ou les colorants liposolubles ou hydrosolubles, par exemple à raison de 0,01 à 70% du poids total de la composition.

Les composés pulvérulents peuvent être choisis parmi les pigments et/ou les nacres et/ou les charges habituellement utilisés dans les compositions cosmétiques ou pharmaceutiques. Avantageusement, les composés pulvérulents représentent 0,1 à 50% du poids total de la composition et mieux de 1 à 40%.

Les pigments peuvent être blancs ou colorés, minéraux et/ou organiques, interférentiels ou non. On peut citer, parmi les pigments minéraux, le dioxyde de titane, éventuellement traité en surface, les oxydes de zirconium ou de cérium, ainsi que les oxydes de fer ou de chrome, le violet de manganèse, le bleu outremer, l'hydrate de chrome et le bleu ferrique. Parmi les pigments organiques, on peut citer le noir de carbone, les pigments de type D & C, et les laques à base de carmin de cochenille, de baryum, strontium, calcium, aluminium.

Les pigments nacrés peuvent être choisis parmi les pigments nacrés blancs tels que le mica recouvert de titane, ou d'oxychlorure de bismuth, les pigments nacrés colorés tels que le mica titane avec des oxydes de fer, le mica titane avec notamment du bleu ferrique ou de l'oxyde de chrome, le mica titane avec un pigment organique du type précité ainsi que les pigments nacrés à base d'oxychlorure de bismuth.

Les charges peuvent être minérales ou organiques, lamellaires ou sphériques. On peut citer le talc, le mica, la silice, le kaolin, les poudres de Nylon et de polyéthylène, de poly-β-alanine et de polyéthylène, le Téflon, la lauroyl-lysine, l'amidon, le nitrure de bore, les poudres de polymères de tétrafluoroéthylène, les microsphères creuses telles que l'Expancel (Nobel Industrie), le polytrap (Dow Corning) et les microbilles de résine de silicone (Tospearls de Toshiba, par exemple), le carbonate de calcium précipité, le carbonate et l'hydro-carbonate de magnésium, l'hydroxyapatite, les microsphères de silice creuses (SILICA BEADS de MAPRECOS), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium.

Les colorants liposolubles sont par exemple le rouge Soudan, le DC Red 17, le DC Green 6, le β-carotène, l'huile de soja, le brun Soudan, le DC Yellow, 11, le DC Violet 2, le DC orange 5, le jaune quinoléine. Ils peuvent représenter de 0 à 20 % et notamment 0,01 à 20 % du poids de la compositions et mieux de 0,1 à 6 %. Les colorants hydrosolubles sont par exemple le jus de betterave, le bleu de méthylène et peuvent représenter jusqu'à 6 % du poids total de la composition.

La composition peut également comprendre des tensioactifs, qui peuvent être choisis parmi les tensioactifs cationiques, anioniques, amphotères et non ioniques, et leurs mélanges. Ces tensioactifs peuvent être présents à raison de 0,01 à 30% en poids, notamment 0,05 à 20% en poids par rapport au poids total de la composition.

La composition peut comprendre en outre tout additif usuellement utilisé dans le domaine cosmétique, tel que des antioxydants, des parfums, des huiles essentielles, des conservateurs, des actifs cosmétiques, des hydratants, des vitamines, des acides gras essentiels, des céramides, des filtres solaires, des polymères, des épaississants, des gélifiants. Bien entendu l'homme du métier veillera à choisir ce ou ces additifs et/ou leur quantité, de manière telle que les propriétés avantageuses de la composition selon l'invention ne soient pas, ou substantiellement pas, altérées par l'adjonction envisagée.

Les compositions selon l'invention peuvent se présenter sous toute forme acceptable et usuelle pour une composition cosmétique ou pharmaceutique.

La composition selon l'invention peut se présenter sous la forme d'une suspension, une dispersion notamment d'huile dans de l'eau grâce à des vésicules; une solution huileuse éventuellement épaissie voire gélifiée; une émulsion huile-dans-eau, eau-dans-huile, ou multiple; un gel ou une mousse; un gel huileux ou émulsionné; une dispersion de vésicules notamment lipidiques; une lotion biphase ou multiphase; un spray; d'une lotion, d'une crème, d'une pommade, d'une pâte souple, d'un onguent, d'un solide coulé ou moulé et notamment en stick ou en coupelle, ou encore de solide compacté.

L'homme du métier pourra choisir la forme galénique appropriée, ainsi que sa méthode de préparation, sur la base de ses connaissances générales, en tenant compte d'une part de la nature des constituants utilisés, notamment de leur solubilité dans le support, et d'autre part de l'application envisagée pour la composition.

La composition cosmétique selon l'invention peut se présenter sous la forme d'un produit de soin et/ou de maquillage de la peau du corps ou du visage, des lèvres et des cheveux, d'un produit solaire ou autobronzant, voire d'un produit capillaire.

Elle trouve notamment une application particulièrement intéressante dans le domaine capillaire, notamment pour le maintien de la coiffure ou la mise en forme des cheveux. Les compositions capillaires sont de préférence des shampooings, des gels, des lotions de mise en plis, des lotions pour le brushing, des compositions de fixation et de coiffage telles que les laques, des mousses ou des sprays. Les lotions peuvent être conditionnées sous diverses formes, notamment dans des vaporisateurs, des flacons-pompe ou dans des récipients aérosol afin d'assurer une application de la composition sous forme vaporisée ou sous forme de mousse.

Les compositions conformes à l'invention peuvent être utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

La composition selon l'invention trouve une autre application particulièrement intéressante dans le domaine du maquillage, plus particulièrement du maquillage des lèvres, des cils et/ou du visage. Elles peuvent donc se présenter avantageusement sous forme de composition de maquillage, notamment de mascara, d'eye-liner, de rouge à lèvres, de fard à joues, de fard à paupières, de fond de teint.

L'invention a encore pour objet un procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie précédemment.

Ce procédé selon l'invention permet notamment le maintien de la coiffure, le traitement, le soin ou le lavage ou le démaquillage de la peau, des cheveux ou de toute autre matière kératinique.

Ce procédé permet également le maquillage de la peau, des cils, des ongles, des cheveux et/ou des lèvres.

L'invention est illustrée plus en détail dans les exemples suivants.

**Exemple 1**

Dans un réacteur de 1 litre, muni d'un réfrigérant, d'une agitation et d'une ampoule d'addition, on introduit les constituants du pied de cuve. On chauffe à 90°C en 1 heure tout en agitant à 150 tr/min. Lorsque la température atteint 90°C, on observe une exothermie d'environ 10°C et un blanchiment. Après retour à 90°C, on ajoute les constituants de la coulée, en 1 heure. On maintient la température à 90°C pendant 3 heures. On distille l'heptane sous pression réduite et on le remplace par de l'isododécane.

| | Pied de cuve (g) | Coulée (g) | Quantité totale |
|---|---|---|---|
| Acrylate de méthyle | 27 | 54 | 81 |
| MPEG 350 * | - | 9 | 9 |
| Amorceur (Trigonox 21 S de Akzo)** | 1,35 | 0,99 | |

(suite)

| | Pied de cuve (g) | Coulée (g) | Quantité totale |
|---|---|---|---|
| Stabilisant (KRATON G1701) | 13,5 | - | |
| Isododécane | 270 | - | |
| Heptane | 103 | - | |
| * MPEG 350 : méthacrylate de méthoxy-poly(éthylène glycol), Bisomer MPEG de Cognis<br>**Trigonox 21 S : tertiobutylperoxy-2-éthyl hexanoate. | | | |

On obtient une dispersion de particules de polymère (90% acrylate de méthyle + 10% MPEG 350) dans l'isododécane.

**Exemple 2**

De façon analogue à l'exemple 1, on prépare la dispersion de polymère suivante :

| | Pied de cuve (g) | Coulée (g) | Quantité totale |
|---|---|---|---|
| Acrylate de méthyle | 27 | 54 | 81 |
| MPEG 550 * | - | 9 | 9 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |
| * MPEG 550 : méthacrylate de méthoxy-poly(éthylène glycol) disponible chez Laporte; Bisomer MPEG de Cognis. | | | |

On obtient une dispersion de particules de polymère (90% acrylate de méthyle + 10% MPEG 550) dans l'isododécane.

**Exemple 3**

De façon analogue à l'exemple 1, on prépare la dispersion de polymère suivante :

| | Pied de cuve (g) | Coulée (g) | Quantité totale |
|---|---|---|---|
| Acrylate de méthyle | 22,5 | 54 | 76,5 |
| Acide acrylique | 4,5 | - | 4,5 |
| MPEG 550 | - | 9 | 9 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 1 | - | 1 |

On obtient une dispersion de particules de polymère (85% acrylate de méthyle + 5% acide acrylique + 10% MPEG 550) dans l'isododécane.

**Exemple 4**

De façon analogue à l'exemple 1, on prépare la dispersion de polymère suivante :

| | Pied de cuve (g) | Coulée (g) | Quantité totale |
|---|---|---|---|
| Acrylate de méthyle | 22,5 | 40.5 | 63 |
| Acide acrylique | 4,5 | - | 4,5 |
| MPEG 550 | - | 9 | 9 |
| Méthacrylate de méthyle | - | 13,5 | 13,5 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (70% acrylate de méthyle + 5% acide acrylique + 15% méthacrylate de méthyle + 10% MPEG 550) dans l'isododécane.

**Exemple 5**

De façon analogue à l'exemple 1, on prépare la dispersion de polymère suivante :

| | Pied de cuve (g) | Coulée (g) | Quantité totale |
|---|---|---|---|
| Acrylate de méthyle | 27 | 36 | 63 |
| MPEG 350 * | - | 27 | 27 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (70% acrylate de méthyle + 30% MPEG 350) dans l'isododécane.

**Exemple 6**

De façon analogue à l'exemple 1, on prépare la dispersion de polymère suivante :

| | Pied de cuve (g) | Coulée (g) | Quantité totale |
|---|---|---|---|
| Acrylate de méthyle | 27 | 36 | 63 |
| MPEG 550 * | - | 27 | 27 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (70% acrylate de méthyle + 30% MPEG 550) dans l'isododécane.

**Exemple 7**

De façon analogue à l'exemple 1, on prépare la dispersion de polymère suivante :

| | Pied de cuve (g) | Coulée (g) | Quantité totale |
|---|---|---|---|
| Acrylate de méthyle | 22,5 | 36 | 58,5 |
| Acide acrylique | 4,5 | - | 4,5 |
| MPEG 550 | - | 27 | 27 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (65% acrylate de méthyle + 5% acide acrylique + 30% MPEG 550) dans l'isododécane.

**Exemple 8**

De façon analogue à l'exemple 1, on prépare la dispersion de polymère suivante :

| | Pied de cuve (g) | Coulée (g) | Quantité totale |
|---|---|---|---|
| Acrylate de méthyle | 22,5 | 16,2 | 38,7 |
| Acide acrylique | 4,5 | - | 4,5 |
| MPEG 550 | - | 27 | 27 |
| Méthacrylate de méthyle | - | 19,8 | 19,8 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (43% acrylate de méthyle + 5% acide acrylique + 22% méthacrylate de méthyle + 30% MPEG 550) dans l'isododécane.

**Exemple 9**

De façon analogue à l'exemple 1, on prépare la dispersion de polymère suivante :

| | Pied de cuve (g) | Coulée (g) | Quantité totale |
|---|---|---|---|
| Acrylate de méthyle | 22,5 | 49,5 | 72 |
| Acide acrylique | 4,5 | 4,5 | 9 |
| MPEG 550 | - | 9 | 9 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (80% acrylate de méthyle + 10% acide acrylique + 10% MPEG 550) dans l'isododécane.

**Exemple 10**

De façon analogue à l'exemple 1, on prépare la dispersion de polymère suivante :

| | Pied de cuve (g) | Coulée (g) | Quantité totale |
|---|---|---|---|
| Acrylate de méthyle | 22,5 | 31,5 | 54 |
| Acide acrylique | 4,5 | 4,5 | 9 |
| MPEG 550 | - | 27 | 27 |
| Amorceur (Trigonox 21 S de Akzo) | 1,35 | 0,99 | |
| Stabilisant (KRATON G1701) | 13,5 | - | |
| Isododécane | 270 | - | |
| Heptane | 103 | - | |

On obtient une dispersion de particules de polymère (60% acrylate de méthyle + 10% acide acrylique + 30% MPEG 550) dans l'isododécane.

**Exemple 11**

1/ préparation du comparatif : dispersion d'acrylate de méthyle/acide acrylique 95/5 % en poids

La synthèse est réalisée en semi-batch dans un réacteur automatisé de 1 litre; on introduit tout d'abord dans le pied de cuve :

- 22,5 g d'acrylate de méthyle (AMe)
- 4,5 g d'acide acrylique (AA)
- 1,35 g de Trigonox 21 S
- 13,5 g de Kraton G1701
- 270 g d'isododécane
- 103 g d'heptane

On chauffe le milieu réactionnel à 90°C en une heure, tout en agitant à 150 tr/min. Lorsque la température du milieu réactionnel atteint 90°C, on assiste à une exotherme d'environ 10°C et un blanchiment. De retour à 90°C, on commence la coulée qui dure environ une heure. Celle-ci contient :

- 63 g d'acrylate de méthyle (AMe)
- 0,99 g de Trigonox 21 S

Une fois la coulée terminée, on laisse polymériser pendant 3 heures à 90°C. On distille ensuite l'heptane, sous pression réduite et on le remplace par de l'isododécane.

2/ évaluation en milieu humide.

Après avoir fait des films de polymère d'environ 1 mm d'épaisseur humide (à partir de dispersion à 20% en poids de polymère) sur le téflon et découpé en carré (3x3 cm), on les immerge dans l'eau à 25°C (0,2 g de film de polymère dans 20 g d'eau) et on suit qualitativement l'évolution de la couleur, du collant, du glissant et de la prise en masse d'eau de ces films. On obtient les résultats suivants (% en poids):

- initialement, sur film sec (avant immersion)
- après 30 minutes d'immersion
- après 14 jours d'immersion
- après 14 jours d'immersion + séchage pendant 24 heures (sur film sec)

| Film | | Comparatif | Ex.1 | Ex.2 | Ex.3 | Ex.4 |
|---|---|---|---|---|---|---|
| Initial (film | Couleur | Translucide bleuté | | | | |
| | Collant | 0 | | | | |

| | | | | | | |
|---|---|---|---|---|---|---|
| sec) | Glissant | 0 | | | | |
| Couleur (après 30 min immersion) | | légèrement trouble | Blanc | Blanc | Blanc | Blanc |
| Im-mergé dans l'eau, 14 jours | Couleur | Trouble | Blanc | Blanc | Blanc | Blanc |
| | Collant (au doigt) | 0 | | | | |
| | Glissant (au doigt) | 0 | 0 | + | + | 0 |
| | Prise de masse (pesée) | 0 | 0 | 0 | + | 0 |
| Film sec : 24 h sé-chage | Couleur | Translucide trouble | | | | |
| | Collant | 0 | | | | |
| | Glissant | 0 | | | | |

| Film | | Comparatif | Ex.5 | Ex.6 | Ex.7 | Ex.8 |
|---|---|---|---|---|---|---|
| Initial (film sec) | Couleur | Translucide bleuté | | | | Translu-cide |
| | Collant | 0 | + | ++ | ++ | 0 |
| | Glissant | 0 | | | | |
| Couleur (30 min après l'immersion) | | Légère-ment trou-ble | Blanc | Blanc | Blanc | Blanc |
| Im-mergé dans l'eau (14 jours) | Couleur | Trouble | Blanc | Blanc | Blanc | Blanc |
| | Collant (au doigt) | 0 | | | | |
| | Glissant (au doigt) | 0 | + | + | ++ | 0 |
| | Prise de masse (pesée) | 0 | 0 | 0 | ++ | + |
| Film sec : 24 h sé-chage | Couleur | Translucide trouble | | | | |
| | Collant | 0 | + | + | + | 0 |
| | Glissant | 0 | | | | |

Collant : « 0 = non collant », « + = un peu collant », « ++ = assez collant»
Glissant : « 0 = pas glissant », « + = un peu glissant », « ++ = assez glissant »
Prise en masse : « 0 = non », « + = un peu », « ++ = assez »

On constate que le film du polymère comparatif est non collant initialement. En milieu humide, il n'est pas glissant, ni collant et on observe ni blanchiment, ni prise en masse.

Les films obtenus avec les dispersions selon l'invention sont très glissants en milieu humide, donc seront plus confortables à porter. Par ailleurs, certains films peuvent absorber/capter l'eau (ils prennent de la masse) et on observe alors un gonflement du film, d'où un volume plus important.

**Exemple 12 : mascara**

On prépare un mascara ayant la composition suivante :

- Cires 25 g
- Amino-2 méthyl-2 propanediol-1,3 0,2 g
- Triéthanolamine 2,4 g
- Acide stéarique 5,4 g
- Diméthicone copolyol (Q2-5220 de DOW-CORNING) 0,2 g
- Polyméthacrylate de sodium 0,25 g MA
- Dispersion de l'exemple 1 0,5 g
- Pigments 6 g
- Conservateurs qs
- Eau qsp 100 g

**Exemple 13 : huile de soin pour le corps**

On prépare une huile hydratante pour peaux sèches à l'aide des constituants suivants :

- triglycérides caprylique/caprique 6,5 g
- propylène glycol dicaprylate/dicaprate 22 g
- octanoate de cétéaryle et myristate d'isopropyle 5 g
- néopentanoate d'isostéaryle 2,5 g
- huile d'arachide 5,25 g
- dispersion de l'exemple 2 0,5 g
- acide palmitique 0,2 g
- antioxydant, conservateurs, parfum qs
- cyclométhicone qsp 100 g

**Exemple 14: composition cosmétique hydratante**

On prépare une composition hydratante comprenant :

Phase A1 :

- Distéarate de sucrose 2,0 %
- Stéarate de sorbitane oxyéthyléné (4OE) 1,4 %
- Acide stéarique 0,75 %
- heptanoate de stéaryle 5,5 %
- Vaseline codex 2,1%
- Huile d'avocat 4,5 %
- Huile de jojoba 4,1 %
- Huile de silicone volatile 3,7 %
- dispersion de l'exemple 3 1 %

Phase A2 :

- Gomme de silicone (Q2-1403 Fluid) 4,0 %
- Parfum, conservateur qs

Phase B :

- conservateur qs
- Triéthanolamine 0,4 %
- Eau qsp 100 %

Phase C :

- Polymères carboxyvinyliques (CARBOPOL 980) 0,3 %
- Eau 9,7 %

Les phases A1 et B sont portées à 65˚C avant d'être associées (B dans A1) sous agitation (rotor-stator). On disperse à température ambiante la phase A2 dans la première dispersion, sous agitation. La phase C, préalablement préparée, est ensuite dispersée afin de gélifier la suspension. On obtient une émulsion hydratante adaptée pour les peaux sèches.

**Exemple 15 : Stick de rouge à lèvres**

La composition de rouge à lèvres suivante est préparée (% en poids):

| | |
|---|---|
| Cire de polyéthylène | 15 % |
| Dispersion de polymère de l'exemple 3 | 10% en MS |
| Huile carbonée non volatile (Parléam) | 26 % |
| Pigments | 8.6 % |
| Isododécane | qsp 100% |

La composition obtenue après application sur les lèvres présente de bonnes propriétés cosmétiques.

**Exemple 16 : Stick de rouge à lèvres**

La composition de rouge à lèvres suivante est préparée (% en poids):

| | |
|---|---|
| Cire de polyéthylène | 15 % |
| Dispersion de polymère de l'exemple 8 | 8% en MS |
| Huile carbonée non volatile (Parléam) | 26 % |
| Pigments | 8,6 % |
| Isododécane | qsp 100% |

La composition obtenue après application sur les lèvres présente de bonnes propriétés cosmétiques.

**Exemple 17 : Fond de teint E/H**

On prépare une composition de fond de teint comprenant les composés suivants :

Phase A

- Cetyl diméthicone copolyol (ABIL EM90) 3 g
- Succinate d'isostéaryl diglycéryle (IMWITOR 780K de la société CONDEA) 0,6 g
- Pigments 10 g
- Isododécane 18,5 g
- Dispersion de polymère de l'exemple 3 8 g en MS

Phase B

- Sulfate de magnésium 0,7 g
- Conservateur qs
- Eau qsp 100 g

Phase C

- Conservateur qs
- Eau 2 g

La composition obtenue présente de bonnes propriétés cosmétiques.

**Revendications**

1. Dispersion de particules d'au moins un polymère éthylénique stabilisé en surface par un agent stabilisant, dans un milieu non aqueux comprenant au moins un composé non aqueux, liquide à 25°C, ayant un paramètre de solubilité global selon l'espace de solubilité de HANSEN inférieur ou égal à 20 $(MPa)^{1/2}$, ou un mélange de tels composés; **caractérisée en ce que** ledit polymère éthylénique comprend de 5% à 50% (exclu) en poids de monomère de formule (I), seul ou en mélange, par rapport au poids total de monomères :

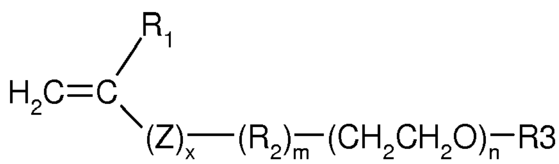

(I)

dans laquelle :

- R1 est un atome d'hydrogène ou un radical hydrocarboné, linéaire ou ramifié, de type $C_pH_{2p+1}$, avec p étant un entier compris entre 1 et 12 inclus;
- Z est un groupement divalent choisi parmi -COO-, -CONH-, $-CONCH_3-$, -OCO- , - O-, $-SO_2-$ -CO-O-CO- ou $-CO-CH_2-CO-$;
- x est 0 ou 1 ;
- R2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m est 0 ou 1 ;
- n est un entier compris entre 3 et 300 inclus;
- R3 est un atome d'hydrogène ou un radical carboné, saturé ou insaturé, éventuellement aromatique, linéaire, ramifié ou cyclique, de 1 à 30 atomes de carbone, pouvant comprendre 1 à 20 hétéroatomes choisis parmi O, N, S, F, Si et P;

et leurs sels.

2. Dispersion selon la revendication 1, dans laquelle R2 est :

- un radical alkylène ayant 1 à 20 atomes de carbone;
- un radical cycloalkylène ayant 5 à 10 atomes de carbone, substitué ou non;
- un radical phénylène $-C_6H_4-$(ortho, méta ou para) éventuellement substitué par un radical alkyle en C1-C12 comprenant éventuellement 1 à 18 hétéroatomes choisis parmi O, N, S, F, Si et P;
- un radical benzylène $-C_6H_4-CH_2-$ éventuellement substitué par un radical alkyle en C1-C12 comprenant éventuellement 1 à 18 hétéroatomes choisis parmi O, N, S, F, Si et P;
- un radical de formule $-CH_2-O-CO-O-$, $CH_2-CH_2-O-CO-O-$, $-CH_2-CO-O-$, $-CH_2-CH_2-CO-O-$, $-CH_2-O-CO-NH-$, $-CH_2-CH_2-O-CO-NH-$; $-CH_2-NH-CO-NH-$, $-CH_2-CH_2-NH-CO-NH-$ ;$-CH_2-CHOH-$, $-CH_2-CH_2-CHOH-$, $-CH_2-CH_2-CH(NH_2)-$, $-CH_2-CH(NH_2)-$, - $CH_2-CH_2-CH(NHR')-$, $-CH_2-CH(NHR')-$, $-CH_2-CH_2-CH(NR'R'')-$, $-CH_2-CH(NR'R'')-$, - $CH_2-CH_2-CH_2-NR'-$, $-CH_2-CH_2-CH_2-O-$; $-CH_2-CH_2-CHR'-O-$ avec R' et R" représentant un alkyle linéaire ou ramifié en C1-C22 comprenant éventuellement 1 à 12 hétéroatomes choisis parmi O, N, S, F, Si et P;
- ou un mélange de ces radicaux.

3. Dispersion selon l'une des revendications précédentes, dans laquelle R3 est un atome d'hydrogène; un radical

phényle éventuellement substitué par un radical alkyle en C1-C12 comprenant éventuellement 1 à 20 hétéroatomes choisis parmi O, N, S, F, Si et P; un radical alkyle en C1-C30 comprenant éventuellement 1 à 18 hétéroatomes choisis parmi O, N, S, F, Si et P; un radical cycloalkyle en C3-C12 comprenant éventuellement 1 à 18 hétéroatomes choisis parmi O, N, S, F, Si et P.

**4.** Dispersion selon l'une des revendications précédentes, dans laquelle le monomère de formule (I) est choisi parmi, seul ou en mélange :

- le (méth)acrylate de poly(éthylène glycol) dans lequel R1 est H ou méthyle; Z est COO, x = 1, m=0 et R3 = H;
- le (méth)acrylate de méthyl-poly(éthylène glycol), aussi appelé (méth)acrylate de méthoxy-poly(éthylèneglycol), dans lequel R1 est H ou méthyle, Z est COO, x = 1, m=0 et R3 = méthyle;
- les (méth)acrylate d'alkyl-poly(éthylène glycol) dans lequel R1 est H ou méthyle, Z est COO, x = 1, m=0 et R3 = alkyl.
- les (méth)acrylates de phényl-poly(éthylène glycol), aussi appelé (méth)acrylate de poly(éthylène glycol) phényl éther, dans lequel R1 est H ou méthyl, Z est COO, x = 1, m=0 et R3 = phényle.

**5.** Dispersion selon l'une des revendications précédentes, dans laquelle le polymère comprend de 7 à 49% en poids, mieux de 8 à 45% en poids, voire de 10 à 40% en poids, notamment de 15 à 35% en poids, de monomère de formule (I), seul ou en mélange, par rapport au poids total de monomères initiaux.

**6.** Dispersion selon l'une des revendications précédentes, dans laquelle le polymère comprend de 50 (inclus) à 95% en poids, par rapport au poids total de monomères, de monomères additionnels, seuls ou en mélange, choisis parmi :

- (i) les monomères de formule (II), et leurs sels:

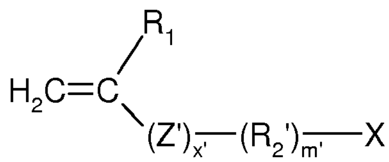

(II)

dans lesquelles :

- R1 est un atome d'hydrogène ou un radical hydrocarboné, linéaire ou ramifié, de type $C_pH_{2p+1}$, avec p étant un entier compris entre 1 et 12, inclus;
- Z' est un groupement divalent choisi parmi -COO- et -CONH-,
- x' est 0 ou 1, de préférence 1.
- R'2 est un radical divalent carboné, saturé ou insaturé, éventuellement aromatique, linéaire, ramifié ou cyclique, de 1 à 30 atomes de carbone, pouvant comprendre 1 à 18 hétéroatomes choisis parmi O, N, S, F, Si et P;
- m' est 0 ou 1 ;
- X est choisi parmi -H, -COOH, $-SO_3H$, $-OSO_3H$, $-PO_3H_2$ et $-OPO_3H_2$.

- (ii) les esters de l'acide (méth)acrylique de formule $CH_2$=CHCOOR4 ou $CH_2=C(CH_3)$COOR4 avec R4 représentant une chaîne carbonée, notamment hydrocarbonée (alkyle), ayant 1 à 32 atomes de carbone, linéaire, cyclique ou ramifiée, saturée ou insaturée, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F).
- (iii) les (méth)acrylamides de formule $CH_2$=CHCONR5R'5 ou $CH_2=C(CH_3)$CONR5R'5 dans lesquelles R5 et R'5, identiques ou différents, représentent un atome d'hydrogène ou une chaîne carbonée, notamment hydrocarbonée (alkyle), ayant 7 à 28 atomes de carbone, linéaire, cyclique ou ramifiée, saturée ou insaturée, éventuellement aromatique, comprenant éventuellement intercalé(s) un ou plusieurs hétéroatomes choisis parmi O, N, S; et/ou éventuellement substituée par un ou plusieurs substituants choisis parmi -OH et les atomes d'halogène (Cl, Br, I et F);
- (iv) les esters vinyliques de formule $CH_2$=CH-OCO-R6 avec R6 représentant une chaîne carbonée, notamment hydrocarbonée, ayant 1 à 12 atomes de carbone, linéaire ou ramifiée, saturée ou insaturée;

- (v) les éthers de vinyle de formule $CH_2$=CHOR7 avec R7 représentant une chaîne carbonée, notamment hydrocarbonée, ayant 2 à 12 atomes de carbone, linéaire ou ramifiée, saturée ou insaturée;
- (vi) les composés vinyliques de formule $CH_2$=CHR8 dans laquelle R8 est

  - un groupe hydroxyle;
  - un groupe alkyle linéaire ou ramifié, comprenant 1 à 25 atomes de carbone, dans lequel se trouve(nt) éventuellement intercalé(s) un ou plusieurs hétéroatomes choisis parmi O, N, S et P; ledit groupe alkyle pouvant, en outre, être éventuellement substitué par un ou plusieurs substituants choisis parmi -OH et les atomes d'halogène (Cl, Br, I et F);
  - un groupe cycloalkyle en $C_3$ à $C_8$ tel que cyclohexane,
  - un groupe aryle en $C_6$ à $C_{20}$ tel que phényle,
  - un groupe aralkyle en $C_7$ à $C_{30}$ (groupe alkyle en $C_1$ à $C_4$) tel que 2-phényléthyle ou benzyle,
  - un groupe hétérocyclique de 4 à 12 chaînons contenant un ou plusieurs hétéroatomes choisis parmi O, N, et S,
  - un groupe hétérocycloalkyle (alkyle de 1 à 4 carbones), tel que furfuryle, furfurylméthyle ou tétrahydrofurfurylméthyle,

lesdits groupes cycloalkyle, aryle, aralkyle, hétérocyclique ou hétérocycloalkyle pouvant être éventuellement substitué par un ou plusieurs substituants choisis parmi le groupe hydroxyle, les atomes d'halogène, et les groupes alkyles en C1-C4, linéaires ou ramifiés dans lesquels se trouve(nt) éventuellement intercalé(s) un ou plusieurs hétéroatomes choisis parmi O, N, S et P, lesdits groupes alkyle pouvant, en outre, être éventuellement substitués par un ou plusieurs substituants choisis parmi -OH, les atomes d'halogène (Cl, Br, I et F).

**7.** Dispersion selon la revendication 6, dans laquelle les monomères additionnels sont choisis parmi, seuls ou en mélange :

- les esters de l'acide (méth)acrylique avec R4 en C1-C18, et notamment les (méth)acrylates de méthyle, d'éthyle, de propyle, de n-butyle, d'isobutyle, de 2-éthylhexyle, de tertiobutyle, d'isooctyle, de décyle, de myristyle, de stéaryle, d'isobornyle,
- le styrène, l'éthylhexylvinylether, le dodécylvinyléther, l'hexanoate de vinyle;
- l'acide acrylique et l'acide méthacrylique.

**8.** Dispersion selon l'une des revendications précédentes, dans laquelle ledit composé liquide non aqueux est choisi parmi :

- les huiles végétales formées par des esters d'acides gras et de polyols, en particulier les triglycérides, telles que l'huile de tournesol, de sésame ou de colza, ou les esters dérivés d'acides ou d'alcools à longue chaîne (ayant de 6 à 20 atomes de carbone), notamment les esters de formule RCOOR' dans laquelle R représente le reste d'un acide gras supérieur comportant de 7 à 19 atomes de carbone et R' représente une chaîne hydrocarbonée comportant de 3 à 20 atomes de carbone, tels que les palmitates, les adipates, les myristates et les benzoates, notamment l'adipate de diisopropyle et le myristate d'isopropyle;
- les hydrocarbures et notamment des huiles de paraffine, de vaseline, ou le polyisobutylène hydrogéné, les isoparaffines en $C_8$-$C_{16}$ et les isoparaffines volatiles tels que l'isododécane ou les 'ISOPARs'.
- les huiles de silicone telles que les polydiméthylsiloxanes et les polyméthylphénylsiloxanes, éventuellement substitués par des groupements aliphatiques et/ou aromatiques, éventuellement fluorés, ou par des groupements fonctionnels tels que des groupements hydroxyles, thiols et/ou amines; et les huiles siliconées volatiles, notamment cycliques ou linéaires, telles que les cyclodiméthylsiloxanes, les cyclophénylmethylsiloxanes et les diméthylsiloxanes linéaires, parmi lesquels on peut citer la dodecamethylpentasiloxane linéaire (L5), l'octaméthylcyclotétrasiloxane, le décaméthylcyclopentasiloxane, l'hexadécaméthylcyclohexasiloxane, l'heptaméthylhexyltrisiloxane, l'heptaméthyloctyltrisiloxane.
- les solvants, seuls ou en mélange, choisis parmi les esters linéaires, ramifiés ou cycliques, ayant 6 à 30 atomes de carbone; les éthers ayant 6 à 30 atomes de carbone et les cétones ayant 6 à 30 atomes de carbone.
- les monoalcools gras aliphatiques ayant au moins 6 atomes de carbone, notamment 6 à 32, la chaîne hydrocarbonée ne comportant pas de groupement de substitution.

**9.** Dispersion selon l'une des revendications précédentes, dans laquelle ledit composé liquide non aqueux est choisi parmi les huiles siliconées volatiles, notamment cycliques ou linéaires, telles que les cyclodiméthylsiloxanes et les diméthylsiloxanes linéaires, et/ou des esters de formule RCOOR' dans laquelle R représente le reste d'un acide

gras supérieur comportant de 7 à 19 atomes de carbone et R’ représente une chaîne hydrocarbonée comportant de 3 à 20 atomes de carbone, tels que les palmitates, les adipates, les myristates et les benzoates, notamment l’adipate de diisopropyle et le myristate d’isopropyle; les hydrocarbures et notamment des huiles de paraffine, de vaseline, ou le polyisobutylène hydrogéné, les isoparaffines en $C_8$-$C_{16}$ et les isoparaffines volatiles tels que l’isododécane ou les ’ISOPARs’. ainsi que leurs mélanges.

**10.** Dispersion selon l’une des revendications précédentes, dans laquelle l’agent stabilisant est présent à raison de 0,1 à 30% en poids par rapport au poids du mélange initial de monomères, de préférence de 1 à 20% en poids, préférentiellement de 2 à 15% en poids, voire de 3 à 10% en poids.

**11.** Dispersion selon l’une des revendications précédentes, dans laquelle l’agent stabilisant est choisi parmi :

- les polymères siliconés greffés avec une chaîne hydrocarbonée et les polymères hydrocarbonés greffés avec une chaîne siliconée.
- les copolymères greffés ayant un squelette insoluble de type polyacrylique avec des greffons solubles de type acide poly(12-hydroxy stéarique).
- les copolymères blocs greffés ou séquencés comprenant au moins un bloc de type polyorganosiloxane et au moins un bloc d’un polymère (i) issu de la polymérisation radicalaire ou (ii) issu d’une polycondensation, notamment de type polyéther, polyester ou polyamide, ou leur mélange, ledit copolymère pouvant comporter des entités fluorées;
- les copolymères blocs, greffés ou séquencés, de (méth)acrylates d’alkyle en C1-C4 et de (méth)acrylates d’alkyle en C8-C30;
- les copolymères blocs greffés ou séquencés comprenant au moins un bloc résultant de la polymérisation de monomère éthylénique, à une ou plusieurs liaisons éthyléniques éventuellement conjuguées, et/ou notamment de diènes; et au moins un bloc de polymère issu de la polymérisation radicalaire autre que diène, notamment issu de monomère vinylique, (meth)acrylique ou (meth)acrylamide, ou d’un polyéther, d’un polyester ou d’un polyamide, ou leurs mélanges;
- les alkyl-dimethicones dans lesquels le groupement alkyl comprend 6 à 32 atomes de carbone, tels que le lauryle methicone, le stéaryle methicone, le cetyl dimethicone, le behenoxydimethicone;
- les dimethiconol esters de formule :

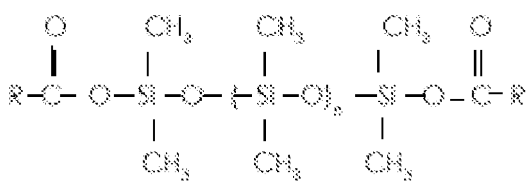

dans laquelle R est un radical alkyle ayant 6 à 32 atomes de carbone, tel que le dimethiconol béhénate,

- les alkylamidoamines ayant notamment 6 à 60 atomes de carbone, notamment 12 à 50, telles que la behenamidopropyldimethylamine.
- les copolymères comprenant au moins une partie polyorganosiloxane et des groupements fluorés, et notamment les silicones fluorées ou fluorosilicones qui peuvent être représentées par la formule :

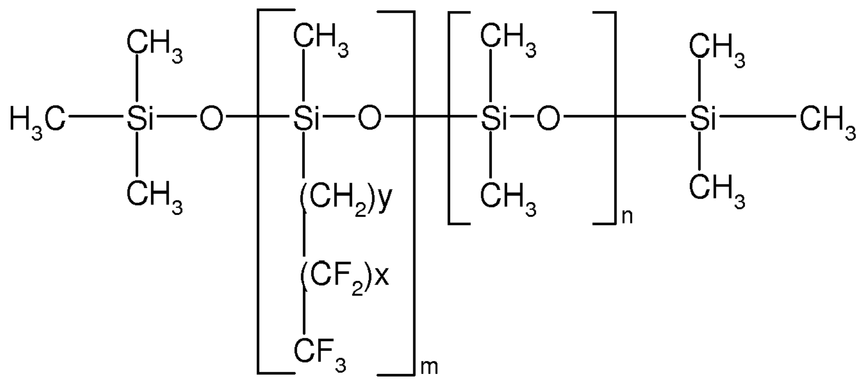

dans laquelle x est un entier compris entre 3 et 12, de préférence 5 et 10, notamment x=8; y est un entier compris entre 2 et 6, de préférence 2 ou 3; et m et n sont tels que le poids moléculaire du composé est compris entre 5000 et 15000.

**12.** Composition cosmétique ou pharmaceutique comprenant, dans un milieu cosmétiquement ou pharmaceutiquement acceptable, au moins une dispersion telle que définie selon l'une des revendications précédentes.

**13.** Composition selon la revendication 12, comprenant par ailleurs au moins un constituant choisi parmi les cires, les huiles, les gommes et/ou les corps gras pâteux, d'origine végétale, animale, minérale ou de synthèse, voire siliconés, et leurs mélanges; une ou plusieurs matières colorantes choisies parmi les composés pulvérulents et/ou les colorants liposolubles ou hydrosolubles; les antioxydants, les parfums, les huiles essentielles, les conservateurs, les actifs cosmétiques, les hydratants, les vitamines, les acides gras essentiels, les céramides, les filtres solaires, les tensioactifs, les polymères, les épaississants, les gélifiants.

**14.** Composition selon l'une des revendications 12 à 13, se présentant sous la forme d'un produit de soin et/ou de maquillage de la peau du corps ou du visage, des lèvres et des cheveux, d'un produit solaire ou autobronzant; d'un produit capillaire notamment pour le traitement, le lavage, le maintien ou la mise en forme des cheveux.

**15.** Composition selon l'une des revendications 12 à 14, se présentant sous la forme d'une composition de maquillage des lèvres, des cils et/ou du visage, notamment de mascara, d'eye-liner, de rouge à lèvres, de fard à joues, de fard à paupières, de fond de teint.

**16.** Procédé de traitement cosmétique des matières kératiniques, notamment de la peau du corps ou du visage, des lèvres, des ongles, des cheveux et/ou des cils, comprenant l'application sur lesdites matières d'une composition cosmétique telle que définie à l'une des revendications 12 à 15.

Europäisches Patentamt
European Patent Office
Office européen des brevets

# RAPPORT DE RECHERCHE EUROPEENNE

Numéro de la demande
EP 09 15 1295

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | EP 0 749 747 A (OREAL [FR]) 27 décembre 1996 (1996-12-27) * le document en entier * ----- | 1-16 | INV. A61K8/72 A61Q1/00 C08J3/09 C08L33/00 |
| A | EP 0 987 012 A (OREAL [FR]) 22 mars 2000 (2000-03-22) * le document en entier * ----- | 1-21 | |
| A | US 2002/151619 A1 (LIN JOHN WEI-PING [US]) 17 octobre 2002 (2002-10-17) * le document en entier * ----- | 1-21 | |
| A | EP 1 256 611 A (JSR CORP [JP]) 13 novembre 2002 (2002-11-13) * le document en entier * ----- | 1-21 | |
| A | US 5 990 202 A (NGUYEN KHE C [US] ET AL) 23 novembre 1999 (1999-11-23) * le document en entier * ----- | 1-21 | |
| | | | DOMAINES TECHNIQUES RECHERCHES (IPC) |
| | | | A61K A61Q C08J C08L |

Le présent rapport a été établi pour toutes les revendications

1

| Lieu de la recherche | Date d'achèvement de la recherche | Examinateur |
|---|---|---|
| Munich | 25 février 2009 | Rouault, Yannick |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
.........................................................................................
& : membre de la même famille, document correspondant

EPO FORM 1503 03.82 (P04C02)

## ANNEXE AU RAPPORT DE RECHERCHE EUROPEENNE RELATIF A LA DEMANDE DE BREVET EUROPEEN NO.

EP 09 15 1295

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

25-02-2009

| Document brevet cité au rapport de recherche | | Date de publication | Membre(s) de la famille de brevet(s) | | | Date de publication |
|---|---|---|---|---|---|---|
| EP 0749747 | A | 27-12-1996 | AT | 174502 | T | 15-01-1999 |
| | | | CA | 2197496 | A1 | 09-01-1997 |
| | | | DE | 69601147 | D1 | 28-01-1999 |
| | | | DE | 69601147 | T2 | 02-06-1999 |
| | | | ES | 2128149 | T3 | 01-05-1999 |
| | | | FR | 2735689 | A1 | 27-12-1996 |
| | | | WO | 9700663 | A1 | 09-01-1997 |
| | | | JP | 2000044426 | A | 15-02-2000 |
| | | | JP | 3225967 | B2 | 05-11-2001 |
| | | | JP | 10501005 | T | 27-01-1998 |
| | | | US | 5851517 | A | 22-12-1998 |
| EP 0987012 | A | 22-03-2000 | BR | 9905295 | A | 20-03-2001 |
| | | | CA | 2281992 | A1 | 18-03-2000 |
| | | | CN | 1249171 | A | 05-04-2000 |
| | | | FR | 2783415 | A1 | 24-03-2000 |
| | | | JP | 2000119124 | A | 25-04-2000 |
| | | | KR | 20000023142 | A | 25-04-2000 |
| | | | US | 6326013 | B1 | 04-12-2001 |
| US 2002151619 | A1 | 17-10-2002 | JP | 2002309144 | A | 23-10-2002 |
| EP 1256611 | A | 13-11-2002 | JP | 2002332354 | A | 22-11-2002 |
| | | | US | 2003073779 | A1 | 17-04-2003 |
| US 5990202 | A | 23-11-1999 | US | 6417249 | B1 | 09-07-2002 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- EP 749747 A

### Littérature non-brevet citée dans la description


- Solubility parameter values. **Grulke.** Polymer Handbook. 519-559
- **HANSEN.** The three dimensionnal solubility parameters. *J. Paint Technol.,* 1967, vol. 39, 105